# EUROPÄISCHE PATENTSCHRIFT

(11) **EP 4 066 728 B1**
(45) Veröffentlichungstag und Bekanntmachung des Hinweises auf die Patenterteilung: **14.08.2024**
(21) Anmeldenummer: 22159359.3
(22) Anmeldetag: 01.03.2022
(51) Int. Cl.: A61B 1/12

(54) **FLUIDVERTEILER FÜR EINE AUFBEREITUNGSEINRICHTUNG ZUM AUFBEREITEN VON CHIRURGISCHEN INSTRUMENTEN**
FLUID DISTRIBUTOR FOR A DEVICE FOR PREPARING SURGICAL INSTRUMENTS
DISTRIBUTEUR DE FLUIDE POUR UN DISPOSITIF DE TRAITEMENT PERMETTANT DE TRAITER DES INSTRUMENTS CHIRURGICAUX

(30) Priorität: 01.04.2021 DE 102021108369
(43) Veröffentlichungstag der Anmeldung: 05.10.2022
(73) Patentinhaber: OLYMPUS WINTER & IBE GMBH, 22045 Hamburg (DE)
(72) Erfinder: HÖRMANN, Mathis Jonathan, 22145 Hamburg (DE)
(74) Vertreter: Seemann & Partner Patentanwälte mbB

(56) Entgegenhaltungen:
- US-A1- 2014 190 520
- US-A1- 2017 367 570
- US-A1- 2020 375 434

## Beschreibung

Die Erfindung betrifft einen Fluidverteiler für eine Aufbereitungseinrichtung zum Aufbereiten eines chirurgischen Instruments, insbesondere Endoskop. Die Erfindung betrifft ferner eine Aufbereitungseinrichtung zum Aufbereiten eines chirurgischen Instruments, insbesondere Endoskop, sowie eine Verwendung eines Fluidverteilers in einer Aufbereitungseinrichtung.

Im Stand der Technik ist bekannt, dass Endoskope zur Diagnose und Therapie von Erkrankungen eingesetzt werden. Für die Verwendung von Endoskopen sowie anderen chirurgischen Geräten ist es notwendig, dass diese chirurgischen Instrumente nach einem Einsatz bei einem Patienten aufbereitet, d.h. gereinigt und desinfiziert, werden müssen.

Bei der Aufbereitung der chirurgischen Instrumente sind dabei gesetzliche sowie klinische Vorschriften streng einzuhalten, so dass die Bestandteile im Inneren, z.B. Endoskopkanäle, und an der Oberfläche der chirurgischen Instrumente, insbesondere Endoskope, desinfiziert werden und frei von Keimen, Bakterien usw. sein müssen.

Aufbereitungseinrichtungen von chirurgischen Instrumenten, insbesondere von Endoskopen, verfügen über Vorrichtungen, die sowohl die äußeren Oberflächen der chirurgischen Instrumente als auch die inneren Kanäle sowie Kanalsysteme unter Verwendung von entsprechenden Flüssigkeiten reinigen und desinfizieren. Hierzu sind üblicherweise Spülkreisläufe vorgesehen. Insbesondere bei der Innenkanalspülung eines chirurgischen Instruments, insbesondere eines Endoskops bzw. eines flexiblen Endoskops, hängt das hygienische Ergebnis in der Aufbereitung von den Durchflussmengen eines Aufbereitungsfluids ab. Als Aufbereitungsfluid können Flüssigkeiten oder Gase dienen.

Darüber hinaus ist zur Aufbereitung und Desinfektion von flexiblen Endoskopen unter der Bezeichnung ETD eine Aufbereitungseinrichtung der Olympus Winter & Ibe GmbH, Hamburg, bekannt. In dieser Aufbereitungseinrichtung werden durch maschinelle Reinigung sowie Desinfektion von, insbesondere flexiblen, Endoskopen die Kanäle der Endoskope mit einem Aufbereitungsfluid bzw. von Spül-, Reinigungs- und/oder Desinfektionsflüssigkeiten durchspült.

Besondere Bedeutung bei der Aufbereitung von chirurgischen Instrumenten, insbesondere Endoskopen, kommt der Reinigung und Desinfektion der Endoskopkanäle zu. Zur Aufbereitung der chirurgischen Instrumente werden hierbei die Kanäle der chirurgischen Instrumente mit einem Spülkreislauf der Aufbereitungseinrichtung verbunden. Zur Aufbereitung der Endoskopkanäle werden die Kanäle z.B. an einem Adapter eines Aufnahmekorbs, in dem das zu reinigende Endoskop angeordnet ist, verbunden, so dass anschließend beim Einbringen des Aufnahmekorbs mit dem zu reinigenden Endoskop in den Spülraum der Aufbereitungseinrichtung die Kanäle mit Spülkanälen des Spülkreislaufs oder der Adapter mit einem Gegenstück des Spülkreislaufs verbunden werden.

Die Ankopplung der Kanäle des Endoskops an einen im Spülraum vorgesehenen Spülkreislauf erfolgt durch Steckkopplungen oder Schraubverbindungen, wobei die Kanäle einzeln und nacheinander mit Leitungen des Spülkreislaufs verbunden werden. Darüber hinaus ist es möglich, dass der Adapter und das Gegenstück miteinander verbunden sind.

Zum Zuführen von Fluiden, insbesondere Flüssigkeiten und Gasen zu den zu reinigenden und/oder zu trocknenden Kanälen existieren in dem Spülkreislauf Abschlussventile, Y-Schlauchverteilungsstücke oder Wegeventile, die aufwendig und oft verlustreich umgeleitet und zusammengeführt werden. Hierbei strömen die Fluide durch Kanäle und können auf mehrere Schläuche verteilt werden.

Ferner ist aus DE 10 2015 203 429 A1 ein Fluidverteiler für eine Aufbereitungseinrichtung zum Aufbereiten von chirurgischen Instrumenten bekannt. Außerdem offenbart DE 10 2011 082 776 A1 eine Vorrichtung zum Spülen von Kanälen eines Endoskops.

In US 2020/0375434 A1 ist ein Ventil für medizinische Vorrichtungen, wie z.B. Endoskope, beschrieben.

Außerdem offenbart US 2014/0190520 A1 eine Spülvorrichtung mit mehreren Spülkanälen, wobei Endoskopkanäle mit jeweils einem Spülkanal verbunden sind.

Weiterhin ist in US 2017/0367570 A1 eine Aufbereitungseinrichtung für chirurgische Instrumente beschrieben, wobei ein Fluidverteiler vorgesehen ist, der mit einer Anschlussplatte verbunden ist.

Es ist Aufgabe der vorliegenden Erfindung, einen einfach aufgebauten Fluidverteiler sowie eine Aufbereitungseinrichtung zum Aufbereiten eines chirurgischen Instruments, insbesondere Endoskop bereitzustellen, wobei der Fluidverteiler flexibel für die Reinigung und/oder Trocknung von mehreren Kanälen bzw. für die Aufbereitung von chirurgischen Instrumenten einsetzbar sein soll.

Gelöst wird diese Aufgabe durch einen Fluidverteiler für eine Aufbereitungseinrichtung zum Aufbereiten eines chirurgischen Instruments, insbesondere Endoskop, mit einem, insbesondere äußeren, Fluidweiterleitungskörper, wobei der Fluidweiterleitungskörper mehrere einzelne Anschlüsse für jeweils einen zu reinigenden und/oder zu trocknenden Kanal eines chirurgischen Instruments aufweist, wobei im Fluidweiterleitungskörper ein, vorzugsweise linear, bewegbarer Fluidschaltkörper vorgesehen ist, wobei der Fluidschaltkörper im Inneren eine mit einem Fluidvorrat verbindbare oder verbundene Zuführleitung für ein, vorzugsweise flüssiges oder gasförmiges, Fluid zu den Anschlüssen des Fluidweiterleitungskörpers aufweist, wobei der bewegbare Fluidschaltkörper derart eingerichtet ist, dass in einer, vorzugsweise ersten, Position des Fluidschaltkörpers im Fluidweiterleitungskörper mehrere oder alle Anschlüsse des Fluidweiterleitungskörpers fluidisch mit der Zuführleitung des Fluidschaltkörpers gleichzeitig verbunden sind, so dass Fluid über die eine Zuführleitung des Fluidschaltkörpers den mehreren oder allen Anschlüssen des Fluidweiterleitungskörpers gleichzeitig zuführbar ist oder zugeführt wird, und dass in wenigstens einer weiteren Position des Fluidschaltkörpers im Fluidweiterleitungskörper, vorzugsweise ausschließlich, einer der mehreren Anschlüsse des Fluidweiterleitungskörpers fluidisch mit der Zuführleitung des Fluidschaltkörpers verbunden ist, so dass das Fluid über die eine Zuführleitung des Fluidschaltkörpers dem einem Anschluss des Fluidweiterleitungskörpers zuführbar ist, der dadurch weitergebildet ist, dass zur Bewegung des Fluidschaltkörpers ein Antrieb für den Fluidschaltkörper vorgesehen ist.

Die Erfindung beruht auf dem Gedanken, dass mittels des erfindungsgemäßen Fluidverteilers wahlweise mehrere oder alle Anschlüsse des Fluidweiterleitungskörpers gleichzeitig fluidisch mit der Zuführleitung des Fluidschaltkörpers verbunden sind oder dass ausschließlich einzelne Anschlüsse des Fluidweiterleitungskörpers fluidisch jeweils mit der Zuführleitung des Fluidschaltkörpers verbunden sind. Somit ist der, vorzugsweise äußere, Fluidweiterleitungskörper als eine Art Fluidverteilungskörper mit mehreren Anschlüssen für die Kanäle des zu reinigenden und/oder zu trocknenden chirurgischen Instruments ausgebildet, so dass je nach Stellung bzw. Position des Fluidschaltkörpers innerhalb des Fluidweiterleitungskörpers die Anschlüsse des Fluidweiterleitungskörpers fluidisch mit der Zuführleitung des Fluidschaltkörpers verbunden bzw. gekoppelt sind. Insbesondere ist es vorgesehen, dass bei n (n = 2, 3, 4, 5...) Anschlüssen am Fluidweiterleitungskörper der Fluidschaltkörper im Inneren des Fluidweiterleitungskörper in (n + 1) Positionen positioniert werden kann bzw. positionierbar ist, um entweder alle Anschlüsse des Fluidweiterleitungskörpers gleichzeitig fluidisch mit der Zuführleitung des Fluidschaltkörpers zu verbinden oder jeweils alle Anschlüsse des Fluidweiterleitungskörpers separat bzw. einzeln fluidisch mit der Zuführleitung des Fluidschaltkörpers zu verbinden.

Im Rahmen der Erfindung ist es möglich, dass für eine Positionsänderung des Fluidschaltkörpers innerhalb des Fluidweiterleitungskörpers der Fluidweiterleitungskörper und der Fluidschaltkörper relativ zueinander bewegt werden oder bewegbar sind.

In einer Position des Fluidschaltkörpers innerhalb des Fluidweiterleitungskörpers wird beispielweise an einem zentralen Hohlzylinder des Fluidweiterleitungskörpers mit nur einem geöffneten Anschluss des Fluidweiterleitungskörpers, der mit einem zu spülenden Kanal eines chirurgischen Instruments über eine Zuführleitung bzw. Spülleitung verbunden ist, das flüssige oder gasförmige Fluid aus einem Fluidvorrat zugeführt, wobei in einer Ausgestaltung die Zuführung des Fluids überwacht wird. Mittels des beweglichen Fluidschaltkörpers, der z.B. als ein bewegliches Rohr ausgebildet ist, wird die Zuführung des Fluids gesteuert, so dass entweder alle Anschlüsse gleichzeitig oder jeweils nur ein Auslass des Fluidweiterleitungskörpers von Fluid durchströmt wird.

Mittels des erfindungsgemäßen Fluidverteilers ist es möglich, die Kanäle eines chirurgischen Instruments variabel zu spülen, wobei die Überwachung der Prozesse bei der Aufbereitungseinrichtung bzw. den Reinigungs- und Desinfektionsvorrichtungen bzw. den Trocknungs- und Aufbewahrungseinrichtungen für chirurgische Instrumente auf einfache Weise verbessert ist.

Bei der Reinigung und/oder Aufbereitung von chirurgischen Instrumenten werden als Fluid für die jeweiligen Prozesse Spülflüssigkeiten und/oder Spülgase je nach durchzuführendem Prozessschritt den Kanälen des chirurgischen Instruments mittels des Fluidverteilers zugeführt. Während der Aufbereitung der chirurgischen Instrumente ist es außerdem in einem Aspekt vorgesehen, dass auch eine Dichtigkeitsprüfung, bei der beispielsweise dem Innenraum den Kanälen eines Endoskops ein Fluid, insbesondere eine Flüssigkeit, zugeführt wird, unter Verwendung des Fluidverteilers durchgeführt wird oder durchführbar ist. Hierbei wird beispielsweise der Druck des Fluids während des Aufbereitungsprozesses bzw. während der Dichtigkeitsprüfung, beispielsweise mittels eines Sensors, überwacht.

Im Rahmen der Erfindung ist es möglich, dass der erfindungsgemäße Fluidverteiler bei einer Trocknungseinrichtung, insbesondere Trockenschrank, und/oder bei einer Aufbewahrungseinrichtung, insbesondere Aufbewahrungsschrank, eingesetzt wird. Hierbei wird beispielsweise trockene Luft bzw. Druckluft als Fluid eingangsseitig über den Fluidschaltkörper des Fluidverteilers zu wenigstens einem der Anschlüsse des Fluidweiterleitungskörpers in eine mit dem Fluidweiterleitungskörper angeschlossene Verbindung zu einem der Kanäle oder den Kanälen in einem chirurgischen Instrument, insbesondere Endoskop, geführt, um den oder die Kanäle des chirurgischen Instruments nach einem Reinigungs- und Desinfektionsprozess zum Beispiel in einer Reinigungs- und Desinfektionseinrichtung zu trocknen. Je nach Stellung bzw. Positionierung des Fluidschaltkörpers im Fluidweiterleitungskörper werden ein Anschluss oder mehrere, insbesondere alle, Anschlüsse des Fluidweiterleitungskörpers mit, vorzugsweise trockener, Luft oder Druckluft beaufschlagt. Bei einer Trocknungseinrichtung, insbesondere Trockenschrank, und/oder Aufbewahrungseinrichtung, insbesondere Aufbewahrungsschrank, für chirurgische Instrumente wird als Prozessfluid ein gasförmiges Fluid, insbesondere trockene Luft oder Druckluft, verwendet.

Darüber hinaus kann im Rahmen der Erfindung der Fluidverteiler auch für Dichtigkeitsmessungen verwendet werden, wobei hierfür der Druckverlauf im Innenraum bzw. die Druckverläufe in den einzelnen Kanälen eines chirurgischen Instruments gemessen wird. Dabei werden unter Verwendung des erfindungsgemäßen Fluidverteilers einzelne Kanäle des chirurgischen Instruments mit einem Fluid, insbesondere Luft, beaufschlagt, um in Abhängigkeit der gemessenen Druckverläufe in den einzelnen Kanälen des chirurgischen Instruments die Dichtigkeit des Instruments zu erfassen bzw. zu bestimmen. Darüber hinaus können auch alle einzelnen Kanäle in einer entsprechenden Position des Fluidschaltkörpers mit einem, insbesondere gasförmigen, Fluid wie zum Beispiel Luft gleichzeitig beaufschlagt werden. Hierdurch wird es möglich, die Dichtigkeit der Kanäle des chirurgischen Instruments bzw. die Dichtigkeit des Innenraums des chirurgischen Instruments zu überwachen.

Darüber hinaus ermöglicht der erfindungsgemäße Fluidverteiler, auf einfache Weise die Blockierung von einzelnen Kanälen des chirurgischen Instruments, insbesondere Endoskops, zum Beispiel bei der Aufbereitung und/oder Trocknung mittels entsprechender Prozessfluide zu erfassen.

Außerdem ist es im Rahmen der Erfindung gemäß einem weiteren Aspekt vorgesehen, dass mittels des Fluidverteilers die Ankopplung von einzelnen oder allen zu spülenden Kanälen des Endoskops zu einem Adapter für die Endoskopkanäle geprüft und/oder überwacht werden kann. Dies kann sowohl bei der Reinigung als auch bei der Trocknung der Endoskopkanäle erfolgen. Dadurch ist es beispielsweise möglich, dass ein Verlust der Konnektivität vom Adapter zum Endoskop auf einfache Weise erfasst oder festgestellt wird.

Dazu ist in einer Weiterbildung des Fluidverteilers vorgesehen, dass der Fluidschaltkörper im Fluidweiterleitungskörper derart bewegbar ist, dass in einer ersten Position des Fluidschaltkörpers im Fluidweiterleitungskörper alle Anschlüsse des Fluidweiterleitungskörpers mit der einen Zuführleitung des Fluidschaltkörpers gleichzeitig, insbesondere parallel, fluidisch verbunden sind und nach Bewegung des Fluidschaltkörpers aus der ersten Position in weitere, vorzugsweise wahlweise, Positionen des Fluidschaltkörpers im Fluidweiterleitungskörper jeweils einer der Anschlüsse des Fluidweiterleitungskörpers für die zu reinigenden und/oder zu trocknenden Kanäle des chirurgischen Instruments mit der einen Zuführleitung des Fluidschaltkörpers einzeln fluidisch verbunden ist.

Um die Anschlüsse des Fluidweiterleitungskörpers auf einfache Weise mit der Zuführleitung des Fluidschaltkörpers fluidisch zu verbinden, ist vorteilhafterweise gemäß einem weiteren Aspekt vorgesehen, dass der Fluidschaltkörper innerhalb des Fluidweiterleitungskörpers linear bewegbar ist. Zur Reinigung bzw. zur Spülung der Kanäle des chirurgischen Instruments, insbesondere Endoskops, die mit den Anschlüssen des Weiterleitungskörpers z.B. über entsprechende Spülleitungen verbunden sind, ist beispielweise der Fluidschaltkörper mit einem Vorrat für ein Prozessfluid, insbesondere eine Flüssigkeit oder ein Gas, verbunden, so dass das Prozessfluid aus dem Vorrat für das Prozessfluid durch die Zuführleitung des Fluidschaltkörpers zu den jeweils geöffneten Anschlüssen des Fluidweiterleitungskörpers geleitet wird.

Dazu ist in einer Ausführungsform vorgesehen, dass die Zuführleitung des Fluidschaltkörpers als ein, vorzugsweise hohlzylindrisches, Rohr oder mit einem im Fluidweiterleitungskörper aufgenommenen, vorzugsweise hohlzylindrischen, Rohrabschnitt ausgebildet ist. Dabei ist die Zuführleitung bzw. das Rohr oder der Rohrabschnitt je nach Stellung bzw. Position des bewegbaren Fluidschaltkörpers wenigstens teilweise im Innenraum des Fluidweiterleitungskörpers aufgenommen.

Um aus einem Vorrat das flüssige oder gasförmige Fluid zu den Anschlüssen des Fluidweiterleitungskörpers zu leiten, ist gemäß einer Weiterbildung des Fluidverteilers vorgesehen, dass im Fluidweiterleitungskörper das Rohr des Fluidschaltkörpers oder der Rohrabschnitt des Fluidschaltkörpers endseitig oder stirnseitig gegenüber dem Inneren des Fluidweiterleitungskörpers unter Verwendung einer Dichtung, insbesondere einer Dichtlippe oder eines O-Rings, abgedichtet ist, wobei insbesondere die Dichtung als ein ringförmiger Kragen ausgebildet ist. Insbesondere ist der ringförmige Kragen als Dichtung am stirnseitigen Auslass des Fluidschaltkörpers ausgebildet.

Darüber hinaus zeichnet sich eine Ausgestaltung des Fluidverteilers dadurch aus, dass das Rohr einen stirnseitigen Auslass im Fluidweiterleitungskörper aufweist oder der Rohrabschnitt einen stirnseitigen Auslass im Fluidweiterleitungskörper aufweist. Durch den Auslass des Rohres bzw. des Rohrabschnitts wird das Fluid zu den Öffnungen der Anschlüsse im Fluidweiterleitungskörper geführt.

Außerdem ist es bei einer Ausgestaltung des Fluidverteilers bevorzugt, dass im Inneren des Fluidweiterleitungskörpers ein mit dem Auslass des Rohrs oder mit dem Auslass des Rohrabschnitts zusammenwirkender und, vorzugsweise linear, bewegbarer, insbesondere scheibenförmiger, Abdichtdeckel vorgesehen ist, wobei insbesondere der Abdichtdeckel mit dem Rohr oder mit dem Rohrabschnitt verbunden ist und in einem vorbestimmten Abstand zum Auslass des Rohrs oder zum Auslass des Rohrabschnitts angeordnet ist. Durch den Abdichtdeckel wird erreicht, dass innerhalb des Fluidweiterleitungskörpers bei einer entsprechenden Positionierung des Fluidschaltkörpers ausschließlich nur ein Anschluss des Fluidweiterleitungskörpers fluidisch mit der Zuführleitung des Fluidschaltkörpers verbunden ist. Dadurch wird ausschließlich ein Anschluss des Fluidweiterleitungskörpers von dem Fluid aus dem Fluidvorrat durchströmt, wobei das Rohr oder der Rohrabschnitt des Fluidschaltkörpers zusammen mit dem Abdichtdeckel gegenüber dem Innenraum des Fluidweiterleitungskörpers abgedichtet ist.

Bei einem bevorzugten Ausführungsbeispiel ist vorgesehen, dass der Abdichtdeckel mit dem Rohr oder dem Rohrabschnitt in einem vorbestimmten Abstand verbunden ist, wobei gleichzeitig bei der Bewegung des Fluidschaltkörpers auch der Abdichtdeckel bewegt wird oder bewegbar ist. Insbesondere ist der Abdichtdeckel über wenigstens einen Abstandshalter mit dem Fluidschaltkörper verbunden. Hierbei ist der Abdichtdeckel in einem vorbestimmten, konstanten Abstand zur den Auslass aufweisenden Stirnseite des Fluidschaltkörpers angeordnet.

Dazu ist bei einer Weiterbildung vorgesehen, dass der Abdichtdeckel gegenüber dem Inneren des Fluidweiterleitungskörpers unter Verwendung einer Dichtung, insbesondere einer Dichtlippe oder eines O-Rings, am umlaufenden Rand des Abdichtdeckels abgedichtet ist. Vorzugsweise weist gemäß einem weiteren Aspekt der Fluidweiterleitungskörper einen längserstreckten, insbesondere zylindrischen oder hohlzylindrischen, Rohrkörper mit einem Innenraum zur Aufnahme des Fluidschaltkörpers und wenigstens eine mit dem Rohrkörper verbundene Strömungskammer zur Aufnahme eines Endes des Fluidschaltkörpers auf, wobei insbesondere der Rohrkörper zwischen zwei Strömungskammern angeordnet ist. Im Rahmen der Erfindung kann hierbei vorgesehen sein, dass bei Anordnung des Fluidschaltkörpers in der Strömungskammer alle Anschlüsse des Weiterleitungskörpers fluidisch mit der Zuführleitung verbunden sind.

Außerdem ist bei einer Ausgestaltung des Fluidverteilers vorgesehen, dass der Rohrkörper entlang seiner Längserstreckung mehrere nebeneinander angeordnete Anschlüsse für jeweils einen Kanal des chirurgischen Instruments, vorzugsweise in äquidistanten Abständen, aufweist und/oder dass die wenigstens eine Strömungskammer einen Anschluss für einen Kanal des chirurgischen Instruments aufweist. Sind beispielsweise zwei Strömungskammern an jeweils einem Ende des Rohrkörpers angeordnet, so ist im Rahmen der Erfindung vorgesehen, dass bezogen auf die Fließrichtung des Fluids die erste Strömungskammer ausschließlich fluidisch mit dem Rohrkörper verbunden ist und eine nach dem Rohrkörper stromabwärtige bzw. ausgangsseitige Strömungskammer einen Anschluss für einen Kanal des chirurgischen Instruments aufweist.

Insbesondere weisen der Innenraum des Rohrkörpers des Fluidweiterleitungskörpers einen Querschnitt und die Strömungskammer einen Innenraum mit einem Querschnitt auf, wobei der Querschnitt des Innenraums des Rohrkörpers kleiner als der parallel dazu ausgerichtete Querschnitt des Innenraums der Strömungskammer ist.

Vorzugsweise ist der Antrieb als mechanischer Antrieb oder als elektrischer Antrieb oder als pneumatischer Antrieb oder als hydraulischer Antrieb ausgebildet.

Außerdem zeichnet sich eine Ausgestaltung des Fluidverteilers dadurch aus, dass eine Positionssensoreinrichtung zur Erfassung wenigstens einer Position oder mehrerer Positionen des Fluidschaltkörpers innerhalb des Fluidweiterleitungskörpers vorgesehen ist, wobei die Positionssensoreinrichtung als ein optischer Sensor oder als ein mechanisch arbeitender Sensor oder als ein elektrischer Sensor ausgebildet ist.

Ferner wird die Aufgabe gelöst durch eine Aufbereitungseinrichtung zum Aufbereiten eines chirurgischen Instruments, insbesondere Endoskop, mit einem erfindungsgemäßen, voranstehend beschriebenen Fluidverteiler. Zur Vermeidung von Wiederholungen wird auf die voranstehenden Ausführungen ausdrücklich verwiesen.

Darüber hinaus wird die Aufgabe gelöst durch die Verwendung eines Fluidverteilers, wie voranstehend beschrieben, in einer Aufbereitungseinrichtung zum Aufbereiten eines chirurgischen Instruments, insbesondere Endoskop. Auf die obigen Ausführungen wird entsprechend verwiesen.

Im Rahmen der Erfindung ist in einer Ausgestaltung die Aufbereitungseinrichtung als eine Reinigungs- und/oder Desinfektionseinrichtung oder als eine Trocknungs- und/oder Aufbewahrungseinrichtung für chirurgische Instrumente, insbesondere Endoskope, ausgebildet.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Im Rahmen der Erfindung sind Merkmale, die mit "insbesondere" oder "vorzugsweise" gekennzeichnet sind, als fakultative Merkmale zu verstehen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine schematische Darstellung eines erfindungsgemäßen Fluidverteilers einer Aufbereitungseinrichtung für ein chirurgisches Instrument;
- Fig. 2: schematisch einen Querschnitt durch den Fluidverteiler und
- Fig. 3: schematisch eine weitere Darstellung des Fluidverteilers im Querschnitt,
- Fig. 4: eine schematische Darstellung eines erfindungsgemäßen Fluidverteilers einer Trocknungs- und Aufbewahrungseinrichtung für ein chirurgisches Instrument.

In den Zeichnungen sind jeweils gleiche oder gleichartige Elemente und/oder Teile mit denselben Bezugsziffern versehen, so dass von einer erneuten Vorstellung jeweils abgesehen wird.

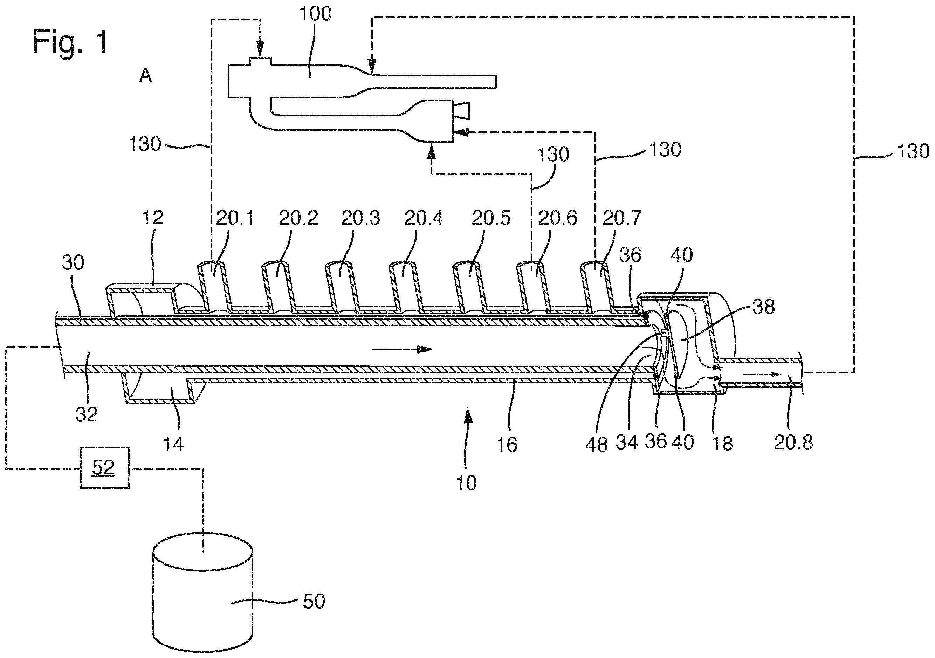

Fig. 1 zeigt einen Fluidverteiler 10 für entsprechende Fluide, wie z.B. Flüssigkeiten oder Gase. Der Fluidverteiler 10 ist Bestandteil einer schematisch bezeichneten Aufbereitungseinrichtung A für ein Endoskop 100. Das Endoskop 100 weist hierbei Kanäle auf, die zu reinigen und zu desinfizieren sind.

Hierzu wird aus einem Spülmittelvorrat 50 mittels einer Pumpe 52 ein Spülfluid dem Fluidverteiler 10 zugefördert. Der Fluidverteiler 10 weist einen äußeren Fluidverteilungskörper 12 auf, der eingangsseitig eine erste Strömungskammer 14 aufweist, die mit einem Rohrzylinder 16 verbunden ist. Ausgangsseitig ist der Rohrzylinder 16 mit einer Strömungskammer 18 verbunden. Die Strömungskammer 18 weist zudem einen Anschluss 20.8 auf, der mit einer Spülleitung 130 für das Endoskop verbunden ist.

Der Rohrzylinder 16 weist entlang seiner Längserstreckung außerdem Anschlüsse 20.1 bis 20.7 auf, die mit entsprechenden Spülleitungen 130 verbunden sind. Exemplarisch zur Illustration sind in Fig. 1 nur vier Spülleitungen 130 eingezeichnet. Die Spülleitungen 130 selbst sind mit entsprechenden Anschlüssen an dem Endoskop 100 versehen, um die entsprechenden (hier nicht dargestellten) Kanäle im Endoskop 100 zu reinigen bzw. zu desinfizieren.

Im Innern des Fluidverteilers 10 ist ein Fluidschaltkörper 30 angeordnet, der innerhalb des Fluidweiterleitungsköpers 12 linear bewegbar ist. Der Fluidschaltkörper 30 ist als hohlzylindrisches Rohr ausgebildet, so dass im Innern des Fluidschaltkörpers 30 eine Zuführleitung 32 ausgebildet ist, die eingangsseitig mit dem Spülmittelvorrat 50 über die Pumpe 52 verbunden. Der Fluidschaltkörper 30 ist zudem eingangsseitig der (ersten) Strömungskammer 14 abgedichtet.

Ausgangsseitig der Zuführleitung 32 ist ein stirnseitiger Auslass 34 ausgebildet, so dass das aus dem Spülmittelvorrat 50 entnommene Spülfluid aus dem Auslass 34 austritt. Im Bereich des stirnseitigen Auslasses 34 ist an der Außenseite eine kragenförmige Dichtung 36 angeordnet, so dass der Auslass 34 der Zuführleitung 32 gegenüber dem Innenraum des Fluidweiterleitungskörpers 12 im Bereich des Rohrzylinders 16 ausgebildet ist.

Ferner ist ausgangsseitig des Auslasses 34 in einem vorbestimmten Abstand ein Abdichtdeckel 38 angeordnet, der ebenfalls mit einer umlaufenden Dichtung 40 an seiner Außenseite ausgebildet ist. Der Abdichtdeckel 38 ist mittels eines Abstandshalters 48 mit der Stirnseite der Zuführleitung 32 fest verbunden.

In Fig. 1 ist der Fluidschaltkörper 30 in einer (End-)Position im Fluidweiterleitungskörper 12 angeordnet, so dass der Auslass 34 der (zweiten) Strömungskammer 18 zugewandt ist und das durch die Zuführleitung 32 geförderte Spülfluid in die Strömungskammer 18 eingebracht wird. Hierbei ist die Strömungskammer 18 im Querschnitt größer als die Querschnittsfläche des Abdichtdeckels 38. Dadurch fließt das Spülmedium durch die Zuführleitung 32 über den Auslass 34 in die Strömungskammer 18, wobei endseitig das Spülmedium ausschließlich über den Auslass 20.8 der damit verbundenen Spülleitung 130 zugeführt wird, wodurch bei der in Fig. 1 dargestellten Position des Fluidschaltkörpers 30 der entsprechend damit verbundene Kanal des Endoskops 100 gespült wird. Die anderen Auslässe 20.1 bis 20.7 werden gemäß der in Fig. 1 gezeigten Position des Fluidschaltköpers 30 nicht mit dem Spülfluid beaufschlagt.

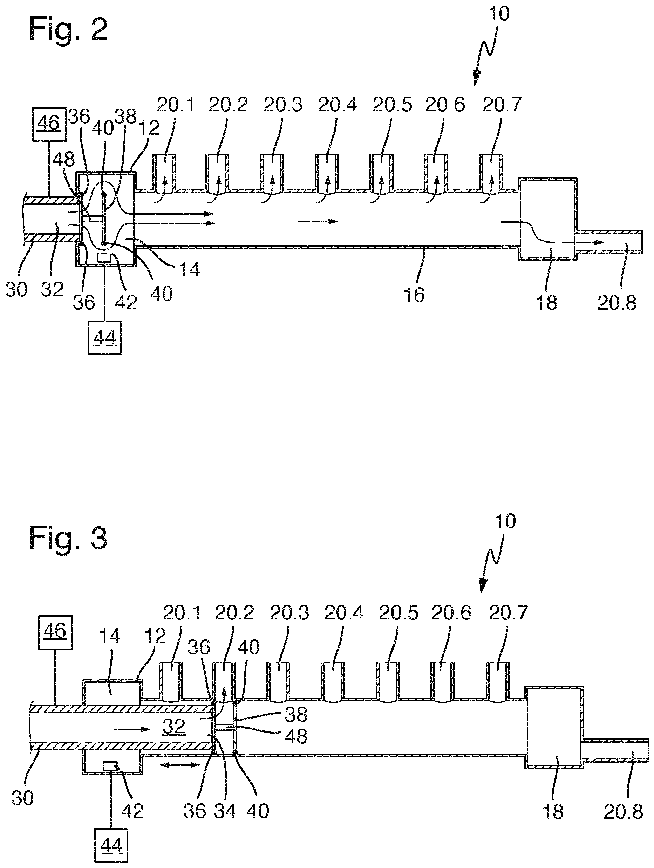

In Fig. 2 und 3 sind weitere Darstellungen des Fluidverteilers 10 gezeigt, wobei hierbei der linear bewegbare Fluidschaltkörper 30 in verschiedenen Positionen dargestellt ist.

Bei der in Fig. 2 gezeigten (Anfangs-)Position des Fluidschaltkörpers 30 ist der Auslass 34 der Zuführleitung 32 innerhalb der ersten Strömungskammer 14 angeordnet, so dass bei Förderung eines Spülfluids das Spülfluid durch sämtliche Auslässe 20.1 bis 20.8 des Fluidweiterleitungskörpers 12 gleichzeitig fließt, so dass sämtliche Kanäle des Endoskops 100 gleichzeitig gespült werden.

Wird der Fluidschaltkörper 30 anschließend linear in Richtung der zweiten Strömungskammer 18 mittels eines Antriebs 46 bewegt, so werden sukzessive in entsprechenden (Zwischen-)Positionen jeweils nur die einzelnen Auslässe 20.1 bis 20.8 jeweils separat von dem Spülmedium durchflossen. Je nach Stellung des Fluidschaltkörpers 30 innerhalb des Fluidweiterleitungskörpers 12 werden hierbei einzeln die Anschlüsse 20.1 bis 20.8 angesteuert, so dass über die einzelnen offenen Anschlüsse 20.1 bis 20.8 das Spülfluid immer nur einem der zu spülenden Anschlüsse 20.1 bis 20.8 und dahinterliegenden bzw. der damit verbundenen Kanäle des Endoskops 100 zugeführt wird.

In Fig. 3 ist der Fluidschaltkörper 30 so positioniert, dass ausschließlich nur der Auslass 20.2 von dem Spülmedium durchflossen wird, während die anderen Auslässe 20.1, 20.3 bis 20.8 nicht von dem Spülmedium durchflossen werden.

Um die Position des Fluidschaltkörpers 30 zu erfassen, ist beispielsweise ein Messsensor 42 in der ersten Strömungskammer 14 angeordnet, wodurch die entsprechende Positionierung des Fluidschaltkörpers 30 innerhalb des Fluidweiterleitungskörpers 12 erfasst wird. Die entsprechenden Messdaten des Messsensors 42 werden hierbei an eine entsprechende Steuerung 44 der Aufbereitungseinrichtung weitergeleitet.

Der Antrieb 46 für den Fluidschaltkörper 30 kann als elektrischer oder hydraulischer oder pneumatischer oder mechanischer Antrieb ausgebildet sein, um den Fluidschaltkörper 30 innerhalb des Fluidweiterleitungskörpers 12 linear entlang der Längserstreckung des Fluidweiterleitungskörpers 12 hin und her zu bewegen.

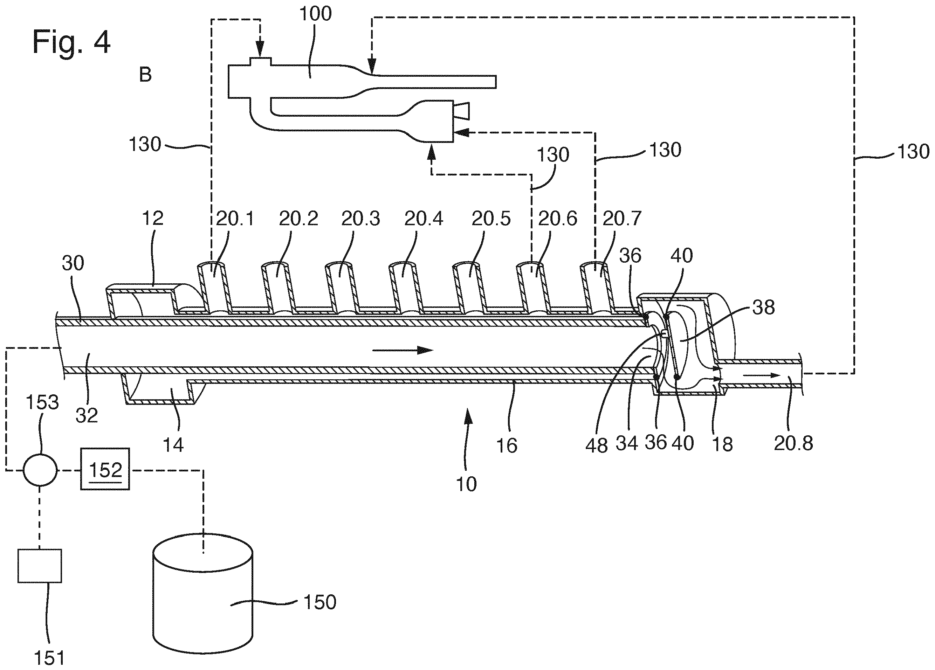

In Fig. 4 ist schematisch als weiteres Ausführungsbeispiel ein Fluidverteiler 10 einer Trocknungs- und Aufbewahrungseinrichtung B für das Endoskop 100 dargestellt. Bei der Trocknungs- und Aufbewahrungseinrichtung B in Fig. 4 ist der Fluidverteiler 10 entsprechend wie in Figur 1 dargestellt ausgeführt, so dass die voranstehenden Ausführungen zum Fluidverteiler 10 in Fig. 1 bis Fig. 3 in analoger Weise hierfür gelten.

Um die einzelnen Kanäle 20.1 bis 20.8 des Fluidweiterleitungskörpers 12 sowie die Kanäle des Endoskops 100 je nach Position des Fluidschaltkörpers mit einem Prozessfluid, insbesondere Luft oder Druckluft, zu trocknen, ist ein Prozessfluidvorrat 150 vorgesehen, der mit dem Fluidschaltkörper 30 bzw. dessen Zuführleitung 32 verbunden ist. In der Verbindung zwischen dem Prozessfluidvorrat 150 und der Zuführleitung 32 sind ein Ventil 152 und ein Sensor 153 angeordnet.

Durch die Öffnung des Ventils 152 wird das Prozessfluid aus dem Prozessfluidvorrat 150 zu dem Auslass 20.8 geleitet. Mittels des Sensors 153 wird der Druck des Prozessfluids in der Leitung gemessen. Der Sensor 153 ist ferner mit einem Dichtigkeitstester 151 verbunden, um die Dichtigkeit des mit dem Auslass 20.8 verbundenen Kanals des Endoskops 100 zu testen. Der Dichtigkeitstester 151 kann hierbei Bestandteil einer Steuereinheit der Trocknungs- und Aufbewahrungseinrichtung B sein.

Bei Positionierung des Fluidschaltkörpers 30 in der Strömungskammer 14 (vergleiche Fig. 2) werden alle mit den Auslässen 20.1 bis 20.8 verbundenen Kanäle des Endoskops 100 mit dem Prozessfluid beaufschlagt. Je nach Positionierung des Fluidschaltkörpers 30 in dem Rohrkörper 16 (vergleiche Fig. 3) wird den einzelnen Kanälen des Endoskops 100 über die entsprechenden Auslässe 20.1 bis 20.8 das Prozessfluid zugeführt.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein.

### Bezugszeichenliste

- 10: Fluidverteiler
- 12: Fluidweiterleitungskörper
- 14: Strömungskammer
- 16: Rohrkörper
- 18: Strömungskammer
- 20.1, 20.8: Auslässe
- 30: Fluidschaltkörper
- 32: Zuführleitung
- 34: Auslass
- 36: Dichtung
- 38: Abdichtdeckel
- 40: Dichtung
- 42: Sensor
- 44: Steuerung
- 46: Antrieb
- 50: Spülmittelvorrat
- 52: Pumpe
- 100: Endoskop
- 130: Spülleitung
- 150: Prozessfluidvorrat
- 151: Dichtigkeitstester
- 152: Ventil
- 153: Sensor

- A: Aufbereitungseinrichtung
- B: Trocknungs- und Aufbewahrungseinrichtung

## Patentansprüche

1. Fluidverteiler (10) für eine Aufbereitungseinrichtung (A) zum Aufbereiten eines chirurgischen Instruments (100), insbesondere Endoskop (100), mit einem, insbesondere äußeren, Fluidweiterleitungskörper (12), wobei der Fluidweiterleitungskörper (12) mehrere einzelne Anschlüsse (20.1, ..., 20.8) für jeweils einen zu reinigenden und/oder zu trocknenden Kanal eines chirurgischen Instruments aufweist, wobei im Fluidweiterleitungskörper (12) ein, vorzugsweise linear, bewegbarer Fluidschaltkörper (30) vorgesehen ist, wobei der Fluidschaltkörper (30) im Inneren eine mit einem Fluidvorrat (50) verbindbare oder verbundene Zuführleitung (32) für ein, vorzugsweise flüssiges oder gasförmiges, Fluid zu den Anschlüssen (20.1, ..., 20.8) des Fluidweiterleitungskörpers (12) aufweist, wobei der bewegbare Fluidschaltkörper (30) derart eingerichtet ist, dass in einer, vorzugsweise ersten, Position des Fluidschaltkörpers (30) im Fluidweiterleitungskörper (12) mehrere oder alle Anschlüsse (20.1, ..., 20.8) des Fluidweiterleitungskörpers (12) fluidisch mit der Zuführleitung (32) des Fluidschaltkörpers (30) gleichzeitig verbunden sind, so dass Fluid über die eine Zuführleitung (32) des Fluidschaltkörpers (30) den mehreren oder allen Anschlüssen (20.1, ..., 20.8) des Fluidweiterleitungskörpers (12) gleichzeitig zuführbar ist oder zugeführt wird, und dass in wenigstens einer weiteren Position des Fluidschaltkörpers (30) im Fluidweiterleitungskörper (12), vorzugsweise ausschließlich, einer der mehreren Anschlüsse (20.1, ..., 20.8) des Fluidweiterleitungskörpers (12) fluidisch mit der Zuführleitung (32) des Fluidschaltkörpers (30) verbunden ist, so dass das Fluid über die eine Zuführleitung (32) des Fluidschaltkörpers (30) dem einem Anschluss des Fluidweiterleitungskörpers (12) zuführbar ist wobei zur Bewegung des Fluidschaltkörpers (30) ein Antrieb (46) für den Fluidschaltkörper (30) vorgesehen ist.

2. Fluidverteiler (10) nach Anspruch 1, **dadurch gekennzeichnet, dass** der Fluidschaltkörper (30) im Fluidweiterleitungskörper (12) derart bewegbar ist, dass in einer ersten Position des Fluidschaltkörpers (30) im Fluidweiterleitungskörper (12) alle Anschlüsse (20.1, ..., 20.8) des Fluidweiterleitungskörpers (12) mit der einen Zuführleitung (32) des Fluidschaltkörpers (30) gleichzeitig, insbesondere parallel, fluidisch verbunden sind und nach Bewegung des Fluidschaltkörpers (30) aus der ersten Position in weitere, vorzugsweise wahlweise, Positionen des Fluidschaltkörpers (30) im Fluidweiterleitungskörper (12) jeweils einer der Anschlüsse (20.1, ..., 20.8) des Fluidweiterleitungskörpers (12) für die zu reinigenden und/oder zu trocknenden Kanäle des chirurgischen Instruments mit der einen Zuführleitung (32) des Fluidschaltkörpers (30) einzeln fluidisch verbunden ist.

3. Fluidverteiler (10) nach Anspruch 1 oder 2, **dadurch gekennzeichnet, dass** der Fluidschaltkörper (30) innerhalb des Fluidweiterleitungskörpers (12) linear bewegbar ist.

4. Fluidverteiler (10) nach einem der Ansprüche 1 bis 3, **dadurch gekennzeichnet, dass** die Zuführleitung (32) des Fluidschaltkörpers (30) als ein, vorzugsweise hohlzylindrisches, Rohr oder mit einem im Fluidweiterleitungskörper (12) aufgenommenen, vorzugsweise hohlzylindrischen, Rohrabschnitt ausgebildet ist.

5. Fluidverteiler (10) nach Anspruch 4, **dadurch gekennzeichnet, dass** im Fluidweiterleitungskörper (12) das Rohr des Fluidschaltkörpers (30) oder der Rohrabschnitt des Fluidschaltkörpers (30) endseitig oder stirnseitig gegenüber dem Inneren des Fluidweiterleitungskörpers (12) unter Verwendung einer Dichtung (36), insbesondere einer Dichtlippe oder eines O-Rings, abgedichtet ist, wobei insbesondere die Dichtung (36) als ein ringförmiger Kragen ausgebildet ist.

6. Fluidverteiler (10) nach Anspruch 4 oder 5, **dadurch gekennzeichnet, dass** das Rohr einen stirnseitigen Auslass (34) im Fluidweiterleitungskörper (12) aufweist oder der Rohrabschnitt einen stirnseitigen Auslass (34) im Fluidweiterleitungskörper (12) aufweist.

7. Fluidverteiler (10) nach einem der Ansprüche 4 bis 6, **dadurch gekennzeichnet, dass** im Inneren des Fluidweiterleitungskörpers (12) ein mit dem Auslass (34) des Rohrs oder mit dem Auslass (34) des Rohrabschnitts zusammenwirkender und, vorzugsweise linear, bewegbarer, insbesondere scheibenförmiger, Abdichtdeckel (38) vorgesehen ist, wobei insbesondere der Abdichtdeckel (38) mit dem Rohr oder mit dem Rohrabschnitt verbunden ist und in einem vorbestimmten Abstand zum Auslass (34) des Rohrs oder zum Auslass (34) des Rohrabschnitts angeordnet ist.

8. Fluidverteiler (10) nach Anspruch 7, **dadurch gekennzeichnet, dass** der Abdichtdeckel (38) gegenüber dem Inneren des Fluidweiterleitungskörpers (12) unter Verwendung einer Dichtung (40), insbesondere einer Dichtlippe oder eines O-Rings, am umlaufenden Rand des Abdichtdeckels abgedichtet ist.

9. Fluidverteiler (10) nach einem der Ansprüche 1 bis 8, **dadurch gekennzeichnet, dass** der Fluidweiterleitungskörper (12) einen längserstreckten, insbesondere zylindrischen oder hohlzylindrischen, Rohrkörper (16) mit einem Innenraum zur Aufnahme des Fluidschaltkörpers (30) und wenigstens eine mit dem Rohrkörper (16) verbundene Strömungskammer (14, 18) zur Aufnahme eines Endes des Fluidschaltkörpers (30) aufweist, wobei insbesondere der Rohrkörper (16) zwischen zwei Strömungskammern (14, 18) angeordnet ist.

10. Fluidverteiler (10) nach Anspruch 9, **dadurch gekennzeichnet, dass** der Rohrkörper (16) entlang seiner Längserstreckung mehrere nebeneinander angeordnete Anschlüsse (20.1, ..., 20.8) für jeweils einen Kanal des chirurgischen Instruments (100), vorzugsweise in äquidistanten Abständen, aufweist und/oder dass die wenigstens eine Strömungskammer (18) einen Anschluss (20.8) für einen Kanal des chirurgischen Instruments (100) aufweist.

11. Fluidverteiler (10) nach Anspruch 9 oder 10, **dadurch gekennzeichnet, dass** der Innenraum des Rohrkörpers (16) des Fluidweiterleitungskörpers (12) einen Querschnitt und die Strömungskammer (18) einen Innenraum mit einem Querschnitt aufweisen, wobei der Querschnitt des Innenraums des Rohrkörpers (16) kleiner als der parallel dazu ausgerichtete Querschnitt des Innenraums der Strömungskammer (18) ist.

12. Fluidverteiler (10) nach einem der Ansprüche 1 bis 11, **dadurch gekennzeichnet, dass** der Antrieb (46) als mechanischer Antrieb oder als elektrischer Antrieb oder als pneumatischer Antrieb oder als hydraulischer Antrieb ausgebildet ist.

13. Fluidverteiler (10) nach einem der Ansprüche 1 bis 12, **dadurch gekennzeichnet, dass** eine Positionssensoreinrichtung (44) zur Erfassung wenigstens einer Position oder mehrerer Positionen des Fluidschaltkörpers (30) innerhalb des Fluidweiterleitungskörpers (12) vorgesehen ist, wobei die Positionssensoreinrichtung (44) als ein optischer Sensor oder als ein mechanisch arbeitender Sensor oder als ein elektrischer Sensor ausgebildet ist.

14. Aufbereitungseinrichtung (A) zum Aufbereiten eines chirurgischen Instruments (100), insbesondere Endoskop (100), mit einem Fluidverteiler (10) nach einem der Ansprüche 1 bis 13.

15. Verwendung eines Fluidverteilers (10) nach einem der Ansprüche 1 bis 13 in einer Aufbereitungseinrichtung (A) zum Aufbereiten eines chirurgischen Instruments (100), insbesondere Endoskop (100).

## Claims

1. A fluid distributor (10) for a reprocessing device (A) for reprocessing a surgical instrument (100), in particular an endoscope (100), with an, in particular outer, fluid routing body (12), wherein the fluid routing body (12) has a plurality of individual connections (20.1, ..., 20.8), each for one channel to be cleaned and/or dried of a surgical instrument, wherein a, preferably linearly, movable fluid switching body (30) is provided in the fluid routing body (12), wherein the fluid switching body (30) has in the interior a supply line (32), connectable or connected to a fluid reservoir (50), for a, preferably liquid or gaseous, fluid to the connections (20.1, ..., 20.8) of the fluid routing body (12), wherein the movable fluid switching body (30) is configured such that, in a, preferably first, position of the fluid switching body (30) in the fluid routing body (12), a plurality of or all connections (20.1, ..., 20.8) of the fluid routing body (12) are fluidically connected to the supply line (32) of the fluid switching body (30) simultaneously, so that fluid can be supplied or is supplied to the plurality of or all connections (20.1, ..., 20.8) of the fluid routing body (12) simultaneously via the one supply line (32) of the fluid switching body (30), and that in at least one further position of the fluid switching body (30) in the fluid routing body (12), preferably exclusively, one of the plurality of connections (20.1, ..., 20.8) of the fluid routing body (12) is fluidically connected to the supply line (32) of the fluid switching body (30) so that the fluid can be supplied to the one connection of the fluid routing body (12) via the one supply line (32) of the fluid switching body (30), wherein a drive (46) for the fluid switching body (30) is provided to move the fluid switching body (30).

2. The fluid distributor (10) according to claim 1, **characterized in that** the fluid switching body (30) is movable in the fluid routing body (12) such that, in a first position of the fluid switching body (30) in the fluid routing body (12), all connections (20.1, ..., 20.8) of the fluid routing body (12) are fluidically connected simultaneously, in particular in parallel, to the one supply line (32) of the fluid switching body (30) and, after the fluid switching body (30) is moved from the first position into other, preferably selectable, positions of the fluid switching body (30) in the fluid routing body (12), one of the connections (20.1, ..., 20.8) of the fluid routing body (12) for each of the channels to be cleaned and/or dried of the surgical instrument is fluidically connected individually to the one supply line (32) of the fluid switching body (30).

3. The fluid distributor (10) according to claim 1 or 2, **characterized in that** the fluid switching body (30) can be moved linearly within the fluid routing body (12).

4. The fluid distributor (10) according to one of claims 1 to 3, **characterized in that** the supply line (32) of the fluid switching body (30) is designed as a, preferably hollow cylindrical, tube or with a, preferably hollow cylindrical, tube portion received in the fluid routing body (12).

5. The fluid distributor (10) according to claim 4, **characterized in that**, in the fluid routing body (12), the tube of the fluid switching body (30) or the tube portion of the fluid switching body (30) is sealed off from the inside of the fluid routing body (12) on the end face or the front face using a seal (36), in particular a sealing lip or an O-ring, wherein in particular the seal (36) is designed as an annular collar.

6. The fluid distributor (10) according to claim 4 or 5, **characterized in that** the tube has a front-face outlet (34) in the fluid routing body (12) or the tube portion has a front-face outlet (34) in the fluid routing body (12).

7. The fluid distributor (10) according to one of claims 4 to 6, **characterized in that**, inside the fluid routing body (12), a, preferably linearly, movable, in particular disk-shaped, sealing cover (38) that interacts with the outlet (34) of the tube or with the outlet (34) of the tube portion is provided, wherein in particular the sealing cover (38) is connected to the tube or to the tube portion and is arranged at a predetermined distance from the outlet (34) of the tube or from the outlet (34) of the tube portion.

8. The fluid distributor (10) according to claim 7, **characterized in that** the sealing cover (38) is sealed off from the inside of the fluid routing body (12) using a seal (40), in particular a sealing lip or an O-ring, on the circumferential edge of the sealing cover.

9. The fluid distributor (10) according to one of claims 1 to 8, **characterized in that** the fluid routing body (12) has an elongated, in particular cylindrical or hollow cylindrical, tube body (16) with an interior for receiving the fluid switching body (30) and at least one flow chamber (14, 18) connected to the tube body (16) for receiving an end of the fluid switching body (30), wherein in particular the tube body (16) is arranged between two flow chambers (14, 18).

10. The fluid distributor (10) according to claim 9, **characterized in that** the tube body (16) has a plurality of connections (20.1, ..., 20.8) arranged next to each other along it longitudinal extension, each for one channel of the surgical instrument (100), preferably at equidistant distances, and/or that the at least one flow chamber (18) has a connection (20.8) for a channel of the surgical instrument (100).

11. The fluid distributor (10) according to claim 9 or 10, **characterized in that** the interior of the tube body (16) of the fluid routing body (12) has a cross-section and the flow chamber (18) has an interior with a cross-section, wherein the cross-section of the interior of the tube body (16) is smaller than the cross-section of the interior of the flow chamber (18), which is aligned parallel to it.

12. The fluid distributor (10) according to one of claims 1 to 11, **characterized in that** the drive (46) is designed as a mechanical drive or as an electric drive or as a pneumatic drive or as a hydraulic drive.

13. The fluid distributor (10) according to one of claims 1 to 12, **characterized in that** a position sensor device (44) is provided for detecting at least one position or a plurality of positions of the fluid switching body (30) within the fluid routing body (12), wherein the position sensor device (44) is designed as an optical sensor or as a mechanically working sensor or as an electrical sensor.

14. A reprocessing device (A) for reprocessing a surgical instrument (100), particularly an endoscope (100), with a fluid distributor (10) according to one of claims 1 to 13.

15. A use of a fluid distributor (10) according to one of claims 1 to 13 in a reprocessing device (A) for reprocessing a surgical instrument (100), particularly an endoscope (100).

## Revendications

1. Distributeur de fluide (10) pour un dispositif de traitement (A) destiné au traitement d'un instrument chirurgical (100), en particulier d'un endoscope (100), avec un corps (12) d'acheminement de fluide, en particulier extérieur, le corps (12) d'acheminement de fluide comportant plusieurs raccords individuels (20.1, ..., 20. 8) pour chaque canal à nettoyer et/ou à sécher d'un instrument chirurgical, un corps déplaçable (30) de commutation de fluide, de préférence linéairement, étant prévu dans le corps (12) d'acheminement de fluide, le corps (30) de commutation de fluide présentant à l'intérieur de lui une conduite d'alimentation (32) apte à être reliée ou est reliée à une réserve de fluide (50) pour un fluide, de préférence liquide ou gazeux, vers les raccords (20. 1, ..., 20.8) du corps (12) d'acheminement de fluide, le corps déplaçable (30) de commutation de fluide étant aménagé de telle sorte que dans une, de préférence première, position du corps (30) de commutation de fluide dans le corps (12) d'acheminement de fluide, plusieurs ou tous les raccords (20. 1, ..., 20.8) du corps (12) d'acheminement de fluide sont reliés simultanément de façon fluidique à la conduite d'alimentation (32) du corps (30) de commutation de fluide, de sorte que du fluide et apte à être acheminé ou est acheminé via ladite une conduite d'alimentation (32) du corps (30) de commutation de fluide vers plusieurs ou vers tous les raccords (20.1, ...., 20.8) du corps (12) d'acheminement de fluide, et que dans au moins une autre position du corps (30) de commutation de fluide dans le corps (12) d'acheminement de fluide, de préférence exclusivement, un raccord de la pluralité de raccords (20. 1, ...., 20.8) du corps (12) d'acheminement de fluide est relié fluidiquement à la conduite d'alimentation (32) du corps (30) de commutation de fluide, de sorte que le fluide est apte à être amené audit un raccord du corps (12) d'acheminement de fluide par ladite une conduite d'alimentation (32) du corps (30) de commutation de fluide, un système d'entraînement (46) pour le corps (30) de commutation de fluide étant prévu pour déplacer le corps (30) de commutation de fluide.

2. Distributeur de fluide (10) selon la revendication 1, **caractérisé en ce que** le corps (30) de commutation de fluide est apte à être déplacé dans le corps (12) d'acheminement de fluide de telle sorte que, dans une première position du corps (30) de commutation de fluide dans le corps (12) d'acheminement de fluide, tous les raccords (20.1, ..., 20. 8) du corps (12) d'acheminement de fluide sont reliés fluidiquement simultanément, en particulier parallèlement, à ladite conduite d'alimentation (32) du corps (30) de commutation et, après le déplacement du corps (30) de commutation de fluide de la première position vers d'autres positions, de préférence au choix, du corps (30) de commutation de fluide dans le corps (12) d'acheminement de fluide, un raccord (20. 1, ..., 20.8) respectif du corps (12) d'acheminement de fluide pour les canaux à nettoyer et/ou à sécher de l'instrument chirurgical est relié individuellement, de façon fluidique, à ladite une conduite d'alimentation (32) du corps (30) de commutation de fluide.

3. Distributeur de fluide (10) selon la revendication 1 ou la revendication 2, **caractérisé en ce que** le corps (30) de commutation de fluide est déplaçable de manière linéaire à l'intérieur du corps (12) d'acheminement de fluide.

4. Distributeur de fluide (10) selon l'une des revendications 1 à 3, **caractérisé en ce que** la conduite d'alimentation (32) du corps (30) de commutation de fluide est réalisée sous la forme d'un tube, de préférence cylindrique creux, ou avec un tronçon de tube, de préférence cylindrique creux, reçu dans le corps (12) d'acheminement de fluide.

5. Distributeur de fluide (10) selon la revendication 4, **caractérisé en ce que** dans le corps (12) d'acheminement de fluide, le tube du corps (30) de commutation de fluide ou le tronçon de tube du corps (30) de commutation de fluide est rendu étanche du côté de l'extrémité ou du côté avant par rapport à l'intérieur du corps (12) d'acheminement de fluide en utilisant un joint (36), en particulier une lèvre d'étanchéité ou un joint torique, le joint (36) étant en particulier réalisé sous la forme d'une collerette annulaire.

6. Distributeur de fluide (10) selon la revendication 4 ou la revendication 5, **caractérisé en ce que** le tube présente une sortie avant (34) dans le corps (12) d'acheminement de fluide ou la section de tube présente une sortie avant (34) dans le corps (12) d'acheminement de fluide.

7. Distributeur de fluide (10) selon l'une des revendications 4 à 6, **caractérisé en ce qu'**à l'intérieur du corps (12) d'acheminement de fluide, se trouve un couvercle d'étanchéité (38) en particulier en forme de disque, coopérant avec la sortie (34) du tube ou avec la sortie du tronçon de tube, et qui est déplaçable, de préférence linéairement, le couvercle d'étanchéité (38) étant notamment relié au tube ou au tronçon de tube et étant agencé à une distance prédéterminée de la sortie (34) du tube ou de la sortie (34) du tronçon de tube.

8. Distributeur de fluide (10) selon la revendication 7, **caractérisé en ce que** le couvercle d'étanchéité (38) est rendu étanche par rapport à l'intérieur du corps (12) d'acheminement de fluide en utilisant un joint d'étanchéité (40), en particulier une lèvre d'étanchéité ou un joint torique, sur le bord périphérique du couvercle d'étanchéité.

9. Distributeur de fluide (10) selon l'une des revendications 1 à 8, **caractérisé en ce que** le corps (12) d'acheminement de fluide présente un corps tubulaire (16) s'étendant longitudinalement, en particulier cylindrique ou cylindrique creux, avec un espace intérieur pour recevoir le corps (30) de commutation de fluide et au moins une chambre d'écoulement (14, 18) reliée au corps tubulaire (16) pour recevoir une extrémité du corps (30) de commutation de fluide, le corps tubulaire (16) étant en particulier disposé entre deux chambres d'écoulement (14, 18).

10. Distributeur de fluide (10) selon la revendication 9, **caractérisé en ce que** le corps tubulaire (16) présente, le long de son extension longitudinale, une pluralité de raccords (20.1, ...., 20.8) agencés les uns à côté des autres, chacun pour un canal de l'instrument chirurgical (100), de préférence à des distances équidistantes, et/ou **en ce que** ladite au moins une chambre d'écoulement (18) présente un raccord (20.8) pour un canal de l'instrument chirurgical (100).

11. Distributeur de fluide (10) selon la revendication 9 ou la revendication 10, **caractérisé en ce que** l'espace intérieur du corps tubulaire (16) du corps (12) d'acheminement de fluide présente une section transversale et la chambre d'écoulement (18) présente un espace intérieur ayant une section transversale, la section transversale de l'espace intérieur du corps tubulaire (16) étant plus petite que la section transversale, orientée parallèlement à celle-ci, de l'espace intérieur de la chambre d'écoulement (18).

12. Distributeur de fluide (10) selon l'une des revendications 1 à 11, **caractérisé en ce que** le système d'entraînement (46) est réalisé sous forme d'un système d'entraînement mécanique ou d'entraînement électrique ou d'entraînement pneumatique ou d'entraînement hydraulique.

13. Distributeur de fluide (10) selon l'une des revendications 1 à 12, **caractérisé en ce qu'**un dispositif (44) de détection de position est prévu pour détecter au moins une position ou plusieurs positions du corps (30) de commutation de fluide à l'intérieur du corps (12) d'acheminement de fluide, le dispositif (44) de détection de position étant conçu sous la forme d'un capteur optique ou d'un capteur fonctionnant mécaniquement ou d'un capteur électrique.

14. Dispositif de traitement (A) pour traiter un instrument chirurgical (100), en particulier un endoscope (100), avec un distributeur de fluide (10) selon l'une des revendications 1 à 13.

15. Utilisation d'un distributeur de fluide (10) selon l'une des revendications 1 à 13 dans un dispositif de traitement (A) pour traiter un instrument chirurgical (100), notamment un endoscope (100).
